# EUROPÄISCHE PATENTSCHRIFT

(11) **EP 0 825 443 B1**
(45) Veröffentlichungstag und Bekanntmachung des Hinweises auf die Patenterteilung: **11.04.2001**
(21) Anmeldenummer: 97111880.7
(22) Anmeldetag: 12.07.1997
(51) Int. Cl.: G01N 33/86

(54) **Verfahren zur Quantifizierung von Glykosaminoglykanen in Antithrombin III-haltigen Lösungen**
Procedure for quantifying glycosaminoglycan in antithrombin III containing solutions
Procédure pour la détermination quantitative de glycosaminoglycan dans une solution contenant de l'antithrombine III

(30) Priorität: 17.08.1996 DE 19633214
(43) Veröffentlichungstag der Anmeldung: 25.02.1998
(73) Patentinhaber: Aventis Behring Gesellschaft mit beschränkter Haftung, 35002 Marburg (DE)
(72) Erfinder: Römisch, Jürgen, Dr., 35041 Marburg (DE); Stauss, Harald, 35232 Dautphetal (DE)

(56) Entgegenhaltungen:
- EP-A- 0 217 768
- EP-A- 0 408 075
- EP-A- 0 657 547
- DATABASE WPI Section Ch, Week 9419 Derwent Publications Ltd., London, GB; Class B04, AN 94-153810 XP002046861 & JP 06 094 713 A (FUNAYAMA M) , 8.April 1994

## Beschreibung

Es wird ein Verfahren beschrieben, das die quantitative Bestimmung von Glykosaminoglykanen (GAG) z. B. Heparin in Antithrombin III-haltigen Lösungen und Lösungen, die andere GAG-bindende Proteine enthalten, ermöglicht.

Antithrombin III ist ein Protein, das zur Familie der Serin-Proteasen-Inhibitoren gehört. Durch Bildung von Komplexen mit Proteasen, wie Thrombin, Gerinnungsfaktor Xa etc. inhibiert AT III die proteolytischen Aktivitäten dieser Enzyme. AT III trägt durch das Spektrum an inhibierenden Proteasen zur Regulation der Hämostase bei. Bei einer Gefäßläsion beispielsweise läßt dieser Inhibitor durch die progressive Art der Inhibition von Gerinnungsfaktoren die Stillung einer Blutung zu; die vollständige Hemmung der aktivierten Gerinnungsproteasen ist erst dann abgeschlossen, wenn der Wundverschluß (Fibrin, Plättchen) erfolgt ist.

Andererseits ist AT III an z.B. das Gefäßendothel über Glykosaminoglykane (GAGs) gebunden und verhindert so eine unkontrollierte Fibrinbildung/Thrombusbildung an der Gefäßwand. Dabei dienen GAGs nicht nur als Rezeptoren, sondern beschleunigen die Reaktion von AT III mit Proteasen. Durch diese nicht-kovalente Wechselwirkung von AT III und z.B. Heparin oder Heparansulfat erfolgt eine Konformationsänderung des Inhibitormoleküles, in der Art, daß eine beschleunigte Komplexbildung mit der Protease ermöglicht wird. Vereinfacht wird dieser Effekt als 'Aktivierung' bezeichnet.

Dieser Akzeleratoreffekt wird therapeutisch genutzt, indem z.B. zur Thromboseprophylaxe Heparin verabreicht wird, das zu den GAGs gerechnet wird. Bei Aktivierung der Gerinnungskaskade, wie sie z.B. häufig bei/nach Operationen auftreten, werden einige der generierten Proteasen durch AT III/Heparin effektiv und schnell inhibiert und so das Risiko einer Thrombusbildung reduziert.

Da im Gegenteil bei einer Überdosierung von Heparin Blutungen auftreten können, müssen der Heparin-Plasmaspiegel des Patienten überwacht und wenn nötig Maßnahmen, wie eine Neutralisierung mit Protamin, ergriffen werden.

Methoden zur Abschätzung der Heparinkonzentration im Plasma werden seit langem angewendet. Diese beruhen z.B. darauf, daß Heparin, z.T. gebunden an AT III oder andere GAG-bindende Proteine, mittels Dextransulfat (DXS) aus der Bindung gedrängt wird. Die nachgeschalteten Detektionssysteme werden im Gegensatz zu Heparin durch DXS in diesen Konzentrationen nicht merklich beeinflußt. Diese Detektionssysteme beruhen wiederum auf der Reaktion des durch DXS liberierten Heparins mit einer definierten Menge AT III, das der Lösung zugesetzt wird. Die durch AT III/Heparin nicht inhibierte Menge der zugesetzten Protease, z.B. Faktor Xa, wird photometrisch mit Hilfe eines chromogenen Substrates bestimmt. Auch koagulometrische Methoden werden angewendet.

Die Heparinmenge von Proben wird an einer Standardkurve, die mit Hilfe von steigenden Heparinmengen erstellt wird, berechnet. Entsprechend therapeutisch sinnvoller und wichtiger Heparinspiegel, werden entsprechende Tests mit einer Sensitivität von lediglich 0,5 bzw. 0.1 IU/ml angeboten.

Antithrombin III-Konzentrate werden dagegen therapeutisch z.B. Patienten verabreicht, die unter einem angeborenen oder erworbenen AT III-Mangel leiden. Da eine Reihe dieser Präparate unter Anwendung immobilisierten Heparins z.B. aus Plasmafraktionen gereinigt werden, ist die Quantifizierung von Heparin in diesen Konzentraten von Interesse. Zur Testung müssen diese Konzentrate möglichst konzentriert eingesetzt werden, um die meist nur in Spuren enthaltenen Heparinmengen überhaupt nachweisen bzw. quantifizieren zu können. Da es sich hierbei um AT III-Konzentrationen von häufig 20 IU/ml und mehr handelt (entsprechend 3 mg/ml AT III und mehr), werden etablierte Testsysteme bereits durch das progressiv-inhibitorische Potential des AT III gestört, was zu falschen Ergebnissen führt. Entsprechend muß dann das Konzentrat soweit verdünnt werden, daß diese Progressivaktivität vernachläßigbar wird. Dies bedeutet wiederum häufig, daß die Heparinspuren mit gebräuchlichen Tests nicht mehr nachweisbar sind. Ein handelsüblicher Assay enthält zum Vergleich 1 IU/ml AT III, mit dessen Hilfe die Heparinkonzentration einer Probe quantifiziert wird.

Aufgabe dieser Erfindung ist es daher, ein Verfahren zur Quantifizierung von Heparin in AT III-haltigen Lösungen bereitzustellen, das unabhängig von der AT III-Konzentration dieser Lösung ist.

Die Aufgabe wird wie folgt gelöst:
1. Die lonenstärke der AT III-haltigen Lösung wird soweit erhöht, daß die Wechselwirkung zwischen AT III und Heparin verhindert wird bzw. Heparin quantitativ aus der nicht-kovalenten Bindung freigesetzt wird. lonenstärken zwischen 15 und 250 mS, bevorzugt zwischen 25 und 150 mS, werden verwendet. Diese werden beispielsweise durch Zugabe entsprechender Mengen von Salzen, z.B. NaCI, bzw. entsprechender Volumina aus hochkonzentrieten Stammlösungen erreicht.
   Dieses Verfahren bietet gegenüber herkömmlichen den Vorteil, daß auf die Zugabe von DXS verzichtet werden kann. Dies ist besonders bei sehr hohen AT III-Konzentrationen vorteilhaft, da in diesen Fällen extreme Mengen an DXS zugegeben werden müßten, die dann das sich anschließende Nachweissystem stören würden.
2. AT III wird aus dieser Lösung entfernt, z.B. mit Hilfe Matrix-gekoppelter Antikörper gegen AT III. In einer bevorzugten Verfahrensweise wird AT III aus der Lösung gefällt und z.B. durch Zentrifugation oder Filtration abgetrennt. Heparin bleibt dabei quantitativ im Überstand. Fällungen können z.B. mit geeigneten Mengen Ammoniumsulfat erfolgen. Bevorzugt wird eine Säure, besonders bevorzugt Trichloressigsäure (0,05M - gesättigt, bevorzugt 0,5-2 M), zur Denaturierung und anschließenden Abtrennung angewendet.
3. Der Überschuß an Säure bzw. Salz wird z.B. durch Dialyse gesenkt, wobei eine starke Verdünnung der Probe verhindert werden muß, um die Detektierbarkeit von Heparinspuren zu gewährleisten. Bevorzugt, da zeitsparend und gut standardisierbar, werden sogenannte Ent-/Umsalzsäulen verwendet, die dem Fachmann als Gelfiltrationen bekannt sind. Der Verdünnungseffekt der Probe ist gering.
4. Die so erhaltene Heparin-haltige Lösung wird in einem geeigneten Testsystem quantifiziert. Darunter versteht man z. B. koagulometrische oder chromogene/fluorigene Assays, die allgemein auf der Kofaktoraktivität der GAGs (s. o.) basieren. Auch immunchemische Tests, wie ELISA, mit gegen die zu bestimmenden GAGs gerichteten Antikörpern, können Verwendung finden. Als weitere Methoden bieten sich chromatographische Techniken, wie HPLC, oder quantitative Elektrophoresen an.

Das beschriebene Verfahren ist nicht auf Heparin beschränkt, sondern schließt ebenso andere GAGs, wie Heparansulfat, Dermatansulfat, Chondroitinsulfat etc. ein.

Auf der anderen Seite kann es auch zur Bestimmung von GAGs verwendet werden, die an andere Proteine als AT III gebunden werden. Dies sind z.B. Heparin-Kofaktor II, Tissue Factor Pathway Inhibitor, Protein C Inhibitor oder Plättchenfaktor 4.

Das Verfahren wird durch folgendes Beispiel erläutert:

### Beispiel:

AT III-Konzentrat wurde mit verschiedenen Mengen an Heparin versetzt. Nach einstündiger Inkubation wurde die Lösung wie folgt behandelt:
1. Gebundenes Heparin wurde durch Zugabe von NaCI aus dem Komplex mit AT III freigesetzt;
2. AT III wurde durch Zugabe von Trichloressigsäure und Zentrifugation entfernt;
3. der Überstand wurde über NAP-5 Gelfiltration entsalzt und die TCA entfernt;
4. das Eluat wurde in einem chromogenen Assay getestet; die Heparinkonzentration wurde mittels einer Standardkurve quantifiziert.

Antithrombin III-Konzentrat (Kybernin^{®}P, Centeon Pharma GmbH) wurde ad 50 IU/ml gelöst und Aliquots mit 0, 20, 50 und 80 mlU/ml Heparin versetzt. Nach einstündiger Inkubation wurden je 0,4 ml dieser Ansätze mit 0,1 ml 5 M NaCI versetzt und für 30 min bei 25°C inkubiert.

Anschließend wurden je 0,3 ml einer 1M Trichloressigsäure zugegeben und die Mischungen für 5 min im Eisbad inkubiert. Nach 2 min Zentrifugation wurden die Überstände (je 0,5 ml) über eine NAP-5 Säule (Fa. Pharmacia Biotech GmbH) abgesäult und die Eluate gesammelt.

Die Quantifizierung des Heparins wurde wie folgt durchgeführt:
0,05 ml Probe oder Standard (Heparin)
0,05 ml AT III Reagenz (0,25 IU/ml AT III, verdünnt in 100 mM
Tris/HCl, pH 7,5, 50 mM NaCl)
*10 min Inkubation bei 25°C*
0,05 ml FXa-Reagenz(Behring Diagnostics; OWLG)
*3 min Inkubation bei 25°C*
0,05 ml FXa-Substrat (Behring Diagnostics; OWLH)
*25 min Inkubation bei 25°C*
0,05 ml 50% Essigsäure

Messung der OD₄₀₅ₙₘ und Auswertung anhand der mitgeführten Heparin-Standardkurve.

Die Heparin-Standardkurve wurde in einem Konzentrationsbereich von 0 bis 200 mIU/ml erstellt ( 0 / 3,125 / 6,25 / 12,5 / 25 / 50 / 100 / 200 mIU/ml Heparin). Die Konzentrationen wurden gegen die Δ OD aufgetragen und die Heparinkonzentrationen anhand dieser Kurve berechnet. Diese Bestimmungen wurden jeweils an drei aufeinander folgenden Tagen durchgeführt.

| **Ergebnis:** | |
|---|---|
| | Heparin (mlU/ml) |
| | Mittelwert ± |
| Standardabweichung | |
| AT III Kontrolle (nicht aufgestockt) | < 10 |
| AT III + 20 mlU/ml Heparin | 24,4±6,3 |
| AT III + 50 mlU/ml Heparin | 49,5±5,3 |
| AT III + 80 mlU/ml Heparin | 82,9±3,8 |

## Patentansprüche

1. Verfahren zur Bestimmung von Glykosaminoglykanen in Antithrombin III-haltigen Lösungen, dadurch gekennzeichnet, daß
a) die Ionenstärke der AT III-haltigen Lösung soweit erhöht wird, daß die Wechselwirkung zwischen AT III und Glykosaminoglykanen verhindert wird,
b) das von Glykosaminoglykanen freigesetzte AT III aus der Lösung entfernt wird,
c) das in Lösung verbliebene Glykosaminoglykan entsalzt und nachgewiesen wird.

2. Verfahren nach Anspruch 1, dadurch gekennzeichnet, daß die Ionenstärke zwischen 15 mS und 250 mS eingestellt wird.

3. Verfahren nach Anspruch 1 oder 2, dadurch gekennzeichnet, daß AT III durch Säurefällung entfernt wird.

4. Verfahren nach Anspruch 1 bis 3, dadurch gekennzeichnet, daß durch Gelfiltration entsalzt wird.

5. Verfahren nach Anspruch 1 bis 4, dadurch gekennzeichnet, daß das Glykosaminoglykan Heparin ist.

## Claims

1. A process for determining glycosaminoglycans in antithrombin III-containing solutions, which comprises
a) increasing the ionic strength of the AT III-containing solution until the interaction between AT III and heparin is prevented,
b) removing the AT III which has been released from glycosaminoglycans from the solution,
c) desalting and detecting the glycosaminoglycan which has remained in solution.

2. The process as claimed in claim 1, wherein the ionic strength is adjusted to between 15 mS and 250 mS.

3. The process as claimed in claim 1, wherein AT III is removed by means of acid precipitation.

4. The process as claimed in claim 1, wherein desalting is carried out by means of gel filtration.

5. The process as claimed in claim 1, wherein the glycosaminoglycan is heparin.

## Revendications

1. Procédé pour la détermination de glycosaminoglycanes dans des solutions contenant de l'antithrombine III, caractérisé en ce que
a) on augmente la force ionique de la solution contenant AT III jusqu'à ce que l'interaction entre AT III et l'héparine soit inhibée,
b) on élimine de la solution l'AT III libérée par les glycosaminoglycanes,
c) on soumet à une élimination des sels le glycosaminoglycane restant en solution et on le détermine.

2. Procédé selon la revendication 1, caractérisé en ce que la force onique est ajustée entre 15 mS et 250 mS.

3. Procédé selon la revendication 1 ou 2, caractérisé en ce que l'AT III est éliminée par précipitation par un acide.

4. Procédé selon l'une des revendications 1 à 3, caractérisé en ce que l'on effectue l'élimination des sels par filtration sur gel.

5. Procédé selon l'une des revendications 1 à 4, caractérisé en ce que le glycosaminoglycane est l'héparine.
